(19) Europäisches Patentamt
European Patent Office
Office européen des brevets

(11) Numéro de publication: **0 322 290 B1**

# (12) FASCICULE DE BREVET EUROPEEN

(45) Date de publication de fascicule du brevet: **12.05.93**

(51) Int. Cl.⁵: **C07C 209/06**, C07C 211/48, C07C 217/84, C07C 323/33

(21) Numéro de dépôt: **88403213.7**

(22) Date de dépôt: **16.12.88**

## (54) Procédé d'allylation des perhalogénoalkyl,perhalogénoalcoxy, perhalogénoalkylthio anilines en présence de métaux.

(30) Priorité: **23.12.87 FR 8718011**

(43) Date de publication de la demande: **28.06.89 Bulletin 89/26**

(45) Mention de la délivrance du brevet: **12.05.93 Bulletin 93/19**

(84) Etats contractants désignés: **AT BE CH DE ES FR GB GR IT LI LU NL SE**

(56) Documents cités:
**EP-A- 0 205 391**
**US-A- 3 642 902**
**US-A- 3 914 311**
**US-A- 4 096 185**

**CHEMICAL ABSTRACTS, vol. 83, no. 1, 7 juillet 1975, page 755, no. 9062t, Columbus, Ohio, US; N.M. MORLYAN et al.: "Copper(I) chloride, a catalyst for substitution of a halogen by amines in allylic halides"**

**(HOUBEN-WEYL) "METHODEN DER ORGANISCHEN CHEMIE", 4. Auflage, vol. XI/I, Stickstoffverbindungen II, 1957, Georg Thieme Verlag, Stuttgart, DE**

(73) Titulaire: **RHONE-POULENC CHIMIE**
**25, quai Paul Doumer**
**F-92408 Courbevoie Cédex(FR)**

(72) Inventeur: **Desmurs, Jean-Roger**
**La Jonquière Route de Ternay**
**F-69360 Communay(FR)**
Inventeur: **Lecouve, Jean-Pierre**
**23E, rue de l'Oratoire**
**F-69300 Caluire(FR)**

(74) Mandataire: **Le Pennec, Magali et al**
**RHONE-POULENC INTERSERVICES Service Brevets Chimie 25, Ouai Paul Doumer**
**F-92408 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

**Description**

La présente invention concerne un procédé de préparation de N-allyl perhalogénoalkyl, perhalogénoalcoxy, perhalogénoalkylthio anilines. Elle concerne plus particulièrement un procédé d'allylation de la métatrifluorométhylaniline en présence de métaux.

Il est connu par exemple selon le brevet US 2 286 678 de condenser le bromure d'allyle sur un aminophénol en présence de carbonate de potassium dans un solvant alcoolique.

Le rendement d'allylation se situe aux environs de 50 %. Ce rendement présente une importance capitale lorsque les matières premières sont très couteuses. Ainsi dans le cas de la métatrifluorométhylaniline on cherche à obtenir des rendements aussi élévés que possible. Dans le cas des aminophénols ce critère est beaucoup moins important.

Il est aussi connu d'après le brevet US 3 668 254 de préparer les N-haloallyl p. phénylène diamines par condensation sur l'amino-4 diphénylamine de dichloro-2,3 propène en présence de quantités stoechiométriques de triéthylamine et en l'absence de solvant.

Les rendements annoncés dans ledit brevet sont toujours très faibles, environ 38 % dans le seul exemple. La technique décrite dans ce brevet n'était donc pas transposable à des matières premières aussi onéreuses que la métatrifluorométhylaniline.

Il faut aussi noter que la métatrifluorométhylaniline est un composé fortement désactivé du point de vue chimique dont la substitution est beaucoup plus difficile que celle des dérivés tels que la paraphénylènediamine qui elle est activée et favorise donc les substitutions.

L'industrie se trouve devant un double problème, obtenir une N-allylation de la métatrifluorométhylaniline avec des rendements corrects sur un produit qui est beaucoup plus difficile à substituer que ceux qui sont décrits dans l'art antérieur.

La présente invention a permis d'atteindre ce double objectif. Elle a pour objet un procédé d'allylation des perhalogénoalkyl, perhalogénoalcoxy, perhalogénoalkylthioanilines
caractérisé en ce que on met en contact en milieu solvant une aniline perhalogénoalkylée, perhalogénoalcoxylée ou perhalogénothioalkylée avec un halogénure d'allyle en présence d'une quantité catalytique d'un métal choisi parmi le palladium, le cuivre et le nickel.

On entend par aniline perhalogénoalkylée, perhalogénoalcoxylée ou perhalogénothioalkylée les composés de formule générale (I) suivante :

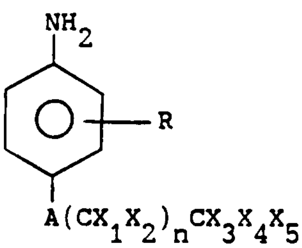 (I)

dans laquelle :

- A représente une liaison covalente, un atome d'oxygène ou de soufre,
- $X_1$, $X_2$, $X_3$, $X_4$, $X_5$ représente chacun un atome d'halogène identique ou différent,
- n est égal à 0,1 ou 2,
- R représente un atome d'hydrogène, un halogène, un groupe alkyle ayant 1 à 4 atomes de carbone, alcoxy ayant 1 à 4 atomes de carbone, aryle.

Ces anilines sont toutes des composés fortement désactivés dont la substitution est difficile.

Il est encore connu d'après le brevet US 3 642 902 de réaliser l'allylation de l'aniline, composé non désactivé, par des complexes du chlorure de bis(allylpalladium). Les rendements d'allylation sont toujours faibles, ils sont de l'ordre de 25 %.

Il est aussi connu d'après les articles de ARESTA parus dans Synth. React. Inorg. Met. Org. Chem., 9-(2), 157-174 de 1979 et dans le Journal of Chemical Society Dalton, (1977), 493 de préparer des complexes du palladium II avec les N-allylanilines. Ces complexes sont facilement détruits avec libération de méthylacétylène et d'un complexe Pd-aniline. A la lecture de ces trois derniers documents, il était impossible d'envisager un procédé industriel mettant en oeuvre des métaux tels que le palladium du fait des risques de formation de ces complexes qui conduisent à une rétrogradation irréversible des N-allylanilines formées à la moindre augmentation de température.

Aussi il a été tout à fait surprenant de constater qu'en mettant en contact une aniline perhalogénoalkylée, perhalogénoalcoxylée ou perhalogénothioalkylée avec un métal choisi parmi le palladium, le nickel et le cuivre il n'y ait aucune dégradation de la N-allylaniline formée et un excellent rendement calculé sur l'aniline transformée.

Le procédé de la présente invention est catalysé comme indiqué précédemment par un métal choisi parmi le palladium, le cuivre et le nickel. Ces métaux peuvent se présenter sous forme de sels métalliques à l'état d'oxydation I ou II notamment de chlorure, de sulfate, d'acétate, d'oxyde. Parmi ces sels, oxydes et complexes on peut citer

- le chlorure de palladium,
- le chlorure cuivreux,
- le chlorure cuivrique,
- le chlorure de nickel,
- le sulfate de palladium,
- le sulfate de cuivre
- le sulfate de nickel,
- l'oxyde cuivrique,
- l'acétate de palladium,
- l'acétate cuivrique,
- le chlorure de palladium complexé par le benzonitrile.

Le métal peut aussi se présenter à l'état d'oxydation O sous forme métallique telle que la poudre de cuivre par exemple ou sous forme de complexe tel que le palladium dibenzylidèneacétone. On préfère parmi les métaux cités utiliser le palladium.

L'halogénure d'allyle est choisi de préférence parmi le chlorure d'allyle et le bromure d'allyle, on préfère encore utiliser le chlorure d'allyle.

La présente invention peut être mise en oeuvre dans les solvants aprotiques polaires tels que :

- la N-méthylpyrrolidone (NMP),
- le N,N-diméthylformamide (DMF),
- le N-méthylformamide (NMF),
- le N,N-diméthylacétamide (DMA),

dans des éthers tels que :

- le tetrahydrofurane (THF),
- l'éther de pétrole,
- le méthyltertiobutyléther,

dans des nitriles tels que :

- le benzonitrile,
- l'acétonitrile,

dans des solvants aromatiques éventuellement halogénés tels que :

- le toluène,
- le xylène,
- le chlorobenzène,
- le dichlorobenzène,

dans des solvants aliphatiques contenant de préférence 5 à 10 atomes de carbone éventuellement halogénés tels que :

- le cyclohexane,
- l'heptane,

dans des alcools et de préférence dans l'éthanol.

Parmi ces solvants on préfère utiliser les solvants aprotiques polaires.

En ce qui concerne les quantités de réactifs mises en oeuvre on préfère utiliser un rapport molaire d'halogénure d'allyle à l'aniline compris entre 1 et 2 et un rapport molaire du catalyseur métallique à l'aniline comprise entre 0,01 et 0,5 de préférence compris entre 0,05 et 0,2.

La concentration de l'aniline dans le solvant est comprise de préférence entre 100 g et 500 g par litre.

En ce qui concerne les conditions réactionnelles on préfère travailler entre 50 et 120 °C pendant une période pouvant évoluer entre 2 et 24 heures.

La présente invention sera plus complètement décrite à l'aide des exemples suivants qui ne doivent en aucun cas être considérés comme limitatifs.

Dans les exemples suivants on entend par :

TT = le taux de transformation du produit initial

$$TT = \frac{\text{nombre de moles de produit transformé}}{\text{nombre de moles de produit initial}}$$

RT = le rendement sur produit transformé

$$RT = \frac{\text{nombre de moles de produit final}}{\text{nombre de moles de produit transformé}}$$

EXEMPLES 1 à 16

Dans un réacteur de 30 ml, on introduit

- 0.65 g de m. trifluorométhylaniline (4 m Moles),
- 0,61 g de chlorure d'alkyle (8 m Moles),
- 2 ml de solvant,
- 0,4 mM de catalyseur.

Le mélange est agité et chauffé à 75° pendant des temps variables. Après refroidissement, on ajoute 5 ml de soude N. Les produits organiques sont extraits par 5 x 5 ml d'éther isopropylique. La phase organique est filtrée sur clarcel et diluée à 25 ml pour être dosée par étalonnage interne en chromatographie phase gazeuse.

| Essai | Catalyseur | Solvant | Temps | TT m-TFMA | RT N-Allyl | RT N,N-Diallyl |
|---|---|---|---|---|---|---|
| Comparatif 1 | Sans | $CH_3CN$ | 2 H 30 | 6,9 | 77,2 | Traces |
| Invention 1 | $PdCl_2$ | $CH_3CN$ | 2 H 30 | 38,6 | 23,5 | |
| Invention 2 | $CuCl_2$ | $CH_3CN$ | 2 H 30 | 66,2 | 26,1 | 43 |
| Invention 3 | $Pd(OAc)_2$ | $CH_3CN$ | 4 H 30 | 78,7 | 38 | 26,2 |
| Invention 4 | $PdCl_2(ØCN)_2$ | $CH_3CN$ | 2 H 30 | 23,7 | 69,6 | 2,5 |
| Invention 5 | $Cu(OAc)_2$ | $CH_3CN$ | 2 H 30 | 11,1 | 91,8 | 8,2 |
| Invention 6 | $Cu_2O$ | $CH_3CN$ | 2 H 30 | 94,0 | 28 | 72 |
| Invention 7 | CuCl | $CH_3CN$ | 3 H 00 | 54,9 | 17,08 | 63,59 |
| Invention 8 | Cu° | $CH_3CN$ | 3 H 00 | 60,5 | 20,9 | 74,2 |
| Invention 9 | $Cu_2O$ | $CH_3CN$ | 3 H 00 | 77,3 | 23,3 | 66,5 |
| Invention 10 | $Pd(OAc)_2$ | $CH_3CN$ | 3 H 00 | 39,9 | 35 | 2,5 |
| Invention 11 | $Pd(OAc)_2$ à 1 % | $CH_3CN$ | 3 H 00 | 12 | 55,8 | 0 |

| Essai | Catalyseur | Solvant | Temps | TT m-TFMA | RT N-Allyl | RT N,N-Diallyl |
|---|---|---|---|---|---|---|
| Comparatif 2 | Sans | EtOH | 2 H 30 | 29,6 | 93,5 | 5 |
| Invention 12 | $Pd(DBA)_2$ | EtOH | 2 H 30 | 72,5 | 39,2 | 30,8 |
| Invention 13 | $Pd(OAc)_2$ | EtOH | 2 H 30 | 57,8 | 43,3 | 14,2 |
| Invention 14 | $NiSO_4$ | EtOH | 2 H 30 | 70,2 | 40,6 | |
| Comparatif 3 | Sans | Heptane | 2 H 30 | 0 | 0 | 0 |
| Invention 15 | $Pd(OAc)_2$ | Heptane | 2 H 30 | 41,9 | 27 | 3,9 |
| Invention 16 | $CuCl_2$ | Heptane | 2 H 30 | 10,5 | 10 | traces |
| Comparatif 4 | Complexe de Cl de diallylpalladium | Heptane | 12 H 30 à 120° | 74 | 0 | 2 |

EXEMPLES 17 à 18

Dans réacteur de 30 ml, on introduit

- 0,65 g de m. trifluorométhylaniline (4m Moles),

- 0,3 g de chlorure d'allyle (4 m Moles),
- 2 ml de solvant,
- 0,4 mM de catalyseur.

Le mélange est chauffé et agité 2 H 30 à 75°. Après refroidissement, on ajoute 5 ml de soude N. Les produits organiques sont extraits par 5 x 5 ml d'éther isopropylique. La phase organique est filtrée sur clarcel et diluée à 25 ml pour être dosée par étalonnage interne.

| Essai | Catalyseur | Solvant | Temps | TT m-TFMA | RT N-Allyl | RT N,N-Diallyl |
|---|---|---|---|---|---|---|
| Invention 17 | $Pd(OAc)_2$ | Heptane | 2 H 30 | 23,7 | 5,7 | |
| Invention 18 | $Cu_2O$ | Heptane | 2 H 30 | 39,3 | 54,1 | 11 % |

EXEMPLES 19 à 23

Dans ces exemples a été étudié l'influence de différents solvants dans les conditions de l'exemple 1.

Influence du solvant

| Essai | Catalyseur | Solvant | Temps | TT m-TFMA | RT N-Allyl | RT N,N-Diallyl |
|---|---|---|---|---|---|---|
| Comparatif 5 | - | Ø-O-Ø | 2 H 30 | 0,3 | Traces | - |
| 19 | Cu | Ø-O-Ø | 2 H 30 | 18,4 | 77 | 16 |
| Comparatif 6 | - | DMF | 2 H 30 | 17,8 | 76,8 | - |
| 20 | Cu | DMF | 2 H 30 | 67,6 | 62,9 | 16,7 |
| Comparatif 7 | - | Toluène | 2 H 30 | 1,1 | Traces | - |
| 21 | Cu | Toluène | 2 H 30 | 21,2 | 69,9 | 5,9 |
| Comparatif 8 | - | NMP | 2 H 30 | 16,9 | 85 | - |
| 22 | Cu | NMP | 2 H 30 | 80,4 | 66,7 | 3 |
| Comparatif 9 | - | DMF - $H_2O$ 2ml - 1ml | 2 H 30 | 74 | 42 | 9,4 |
| 23 | Cu | DMF - $H_2O$ 2ml - 1ml | 2 H 30 | 95,6 | 15,9 | 44,5 |

## Revendications

1. Procédé de préparation de N-allylperhalogénoalkyl, - perhalogénoalcoxy, perhalogénoalkylthio anilines caractérisé en ce que on met en contact une aniline perhalogénoalkylée, - perhalogénoalcoxylée, perhalogénothioalkylée en milieu solvant avec un halogénure d'allyle en présence d'une quantité catalytique d'un métal choisi parmi le palladium, le cuivre et le nickel.

2. Procédé selon la revendication 1 caractérisé en ce que l'aniline répond à la formule générale (I) suivante :

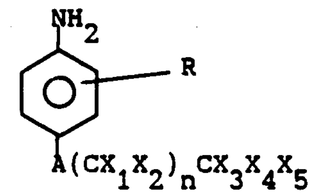

(I)

dans laquelle
   - A représente une liaison covalente, un atome d'oxygène ou de soufre,
   - $X_1,X_2,X_3,X_4,X_5$ représente chacun un atome d'halogène identique ou différent.
   - n est égal à 0,1 ou 2,
   - R représente un atome d'hydrogène, un halogène, un groupe alkyle ayant 1 à 4 atomes de carbone, alcoxy ayant 1 à 4 atomes de carbone, aryle.

3. Procédé selon la revendication 1 caractérisé en ce que ladite aniline est représentée par une trifluorométhylaniline.

4. Procédé selon la revendication 3 caractérisé en ce que la trifluorométhylaniline est la métatrifluorométhylaniline.

5. Procédé selon la revendication 1 caractérisé en ce que l'halogénure d'allyle est choisi parmi le chlorure et le bromure d'allyle et est de préférence le chlorure d'allyle.

6. Procédé selon la revendication 1 caractérisé en ce que le métal est le palladium.

7. Procédé selon les revendications 1 et 5 caractérisé en ce que le catalyseur est choisi parmi le diacétate de palladium et le palladium dibenzylidène acétone.

8. Procédé selon la revendication 1 caractérisé en ce que le solvant est choisi parmi l'heptane et l'acétonitrile.

9. Procédé selon la revendication 1 caractérisé en ce que le rapport molaire de l'halogénure d'allyle à ladite aniline est compris entre 1 et 2.

10. Procédé selon la revendication 1 caractérisé en ce que la température réactionnelle est comprise entre 50 et 120 ° C.

11. Procédé selon la revendication 4 caractérisé en ce que le rapport molaire du catalyseur métallique à la N-métatrifluorométhylaniline est compris entre 0,01 et 0,5 et de préférence entre 0,05 et 0,2.

## Claims

1. Process for the preparation of N-allylperhaloalkyl-, -perhaloalkoxy- and -perhaloalkylthioanilines, characterised in that a perhaloalkylated, perhaloalkoxylated or perhalothioalkylated aniline is placed in a solvent medium in contact with an allyl halide in the presence of a catalytic quantity of a metal chosen from palladium, copper and nickel.

**2.** Process according to Claim 1, characterised in that the aniline corresponds to the following general formula (I):

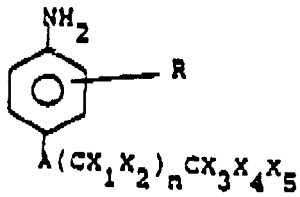

(I)

in which

- A denotes a covalent bond or an oxygen or sulphur atom,
- $X_1$,$X_2$,$X_3$,$X_4$ and $X_5$ each denote an identical or different halogen atom,
- n is equal to 0, 1 or 2, and
- R denotes a hydrogen atom, a halogen or an alkyl containing 1 to 4 carbon atoms, alkoxy containing 1 to 4 carbon atoms or aryl group.

**3.** Process according to Claim 1, characterised in that the said aniline is represented by a trifluoromethylaniline.

**4.** Process according to claim 3, characterised in that the trifluoromethylaniline is meta-trifluoromethylaniline.

**5.** Process according to Claim 1, characterised in that the allyl halide in chosen from allyl chloride and bromide and is preferably allyl chloride.

**6.** Process according to Claim 1, characterised in that the metal is palladium.

**7.** Process according to Claims 1 and 5, characterised in that the catalyst is chosen from palladium diacetate and dibenzylideneacetonepalladium.

**8.** Process according to Claim 1, characterised in that the solvent is chosen from heptane and acetonitrile.

**9.** Process according to Claim 1, characterised in that the molar ratio of the allyl halide to said aniline is between 1 and 2.

**10.** Process according to Claim 1, characterised in that the reaction temperature is between 50 and 120 ° C.

**11.** Process according to Claim 4, characterised in that the molar ratio of the metal catalyst to N-meta-trifluoromethylaniline is between 0.01 and 0.5 and preferably between 0.05 and 0.2.

**Patentansprüche**

**1.** Verfahren zur Herstellung von N-Allylperhalogenalkyl-, -perhalogenalkoxy-, -perhalogenalkylthio-anilinen, dadurch gekennzeichnet, daß man ein Perhalogenalkyl-, Perhalogenalkoxy-, Perhalogenthioalkyl-anilin in einem Lösungsmittelmilieu mit einem Allylhalogenid in Gegenwart einer katalytischen Menge eines Metalls, ausgewählt unter Palladium, Kupfer und Nickel, in Kontakt bringt.

**2.** Verfahren gemäß Anspruch 1, dadurch gekennzeichnet, daß das Anilin der folgenden allgemeinen Formel (I) entspricht:

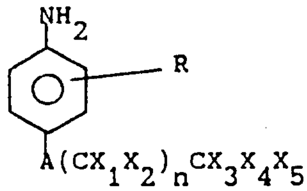

(I)

worin

- A eine kovalente Bindung, ein Sauerstoff- oder Schwefelatom bedeutet,
- $X_1$, $X_2$, $X_3$, $X_4$, $X_5$ jeweils ein identisches oder verschiedenes Halogenatom bedeuten,
- n = 0, 1 oder 2 ist,
- R ein Wasserstoffatom, ein Halogenatom, eine Alkylgruppe mit 1 bis 4 Kohlenstoffatomen, Alkoxy mit 1 bis 4 Kohlenstoffatomen oder Aryl bedeutet.

**3.** Verfahren gemäß Anspruch 1, dadurch gekennzeichnet, daß das genannte Anilin dargestellt ist durch ein Trifluormethylanilin.

**4.** Verfahren gemäß Anspruch 3, dadurch gekennzeichnet, daß das Trifluormethylanilin das Metatrifluormethylanilin ist.

**5.** Verfahren gemäß Anspruch 1, dadurch gekennzeichnet, daß das Allylhalogenid ausgewählt ist unter Allylchlorid und Allylbromid, und vorzugsweise das Allylchlorid ist.

**6.** Verfahren gemäß Anspruch 1, dadurch gekennzeichnet, daß das Metall Palladium ist.

**7.** Verfahren nach den Ansprüchen 1 und 5, dadurch gekennzeichnet, daß der Katalysator ausgewählt ist unter Palladiumdiacetat und Palladiumdibenzylidenaceton.

**8.** Verfahren gemäß Anspruch 1, dadurch gekennzeichnet, daß das Lösungsmittel ausgewählt ist aus Heptan und Acetonitril.

**9.** Verfahren gemäß Anspruch 1, dadurch gekennzeichnet, daß das Molverhältnis des Allylhalogenids zu dem genannten Anilin zwischen 1 und 2 beträgt.

**10.** Verfahren gemäß Anspruch 1, dadurch gekennzeichnet, daß die Reaktionstemperatur zwischen 50 und 120 °C liegt.

**11.** Verfahren gemäß Anspruch 4, dadurch gekennzeichnet, daß das Molverhältnis des metallischen Katalysators zum N-Metatrifluormethylanilin zwischen 0,01 und 0,5 und vorzugsweise zwischen 0,05 und 0,2 beträgt.